# EUROPEAN PATENT SPECIFICATION

(11) **EP 1 838 672 B1**
(45) Date of publication and mention of the grant of the patent: **18.02.2015**
(21) Application number: 05855288.6
(22) Date of filing: 22.12.2005
(51) Int. Cl.: C07D 211/00, C07D 405/00, A61K 31/44

(54) **4-PIPERIDINOL TERTIARY AMINES AS HISTAMINE 3 RECEPTOR INHIBITORS FOR THE TREATMENT OF OBESITY AND OTHER CNS DISORDERS**
TERTIÄRE 4-PIPERIDINOLAMINE ALS INHIBITOREN DES HISTAMIN-3-REZEPTORS ZUR BEHANDLUNG VON OBESITAS UND ANDEREN ZNS-ERKRANKUNGEN
AMINES TERTIAIRES DE 4-PIPERIDINOL EN TANT QU'INHIBITEURS DU RECEPTEUR 3 DE L'HISTAMINE DESTINEES AU TRAITEMENT DE L'OBESITE ET D'AUTRES TROUBLES DU SYSTEME NERVEUX CENTRAL

(30) Priority: 23.12.2004 US 639015 P
(43) Date of publication of application: 03.10.2007
(73) Proprietor: Athersys, Inc., Cleveland, OH 44115-2634 (US)
(72) Inventor: BENNANI, Youssef, Shaker Heights, Ohio 44122 (US); CAMPBELL, Michael, G., Sagamore Hills, Ohio 44067 (US); WANG, Jianmin, Blacksburg, VA 24060 (US); ANDERSON, James, T., Shaker Heights, Ohio 44120 (US)
(74) Representative: Modiano, Micaela Nadia
(86) International application number: PCT/US2005/046703
(87) International publication number: WO 2006/071750

(56) References cited:
- WO-A1-03/031432
- WO-A1-2005/066161
- WO-A2-02/12190
- PROSTAKOV N S ET AL: "1, 2, 5-Trimethyl-4-phenylethynylpiperidol-4 and its reactions" CHEMISTRY OF HETEROCYCLIC COMPOUNDS (A TRANSLATION OF KHIMIYAGETEROTSIKLICHESKIKH SOEDINENII), PLENUM PRESS CO., NEW YORK, NY, US LNKD- DOI:10.1007/BF00472693, vol. 1, no. 6, 1 November 1966 (1966-11-01), pages 576-581, XP008124642 ISSN: 0009-3122 [retrieved on 2004-12-06]
- PROSTAKOV N S ET AL: "Condensation of 1,2,5-trimethyl-4-piperidone with ethyl- and naphthylacetylenes and synthesis of substituted pyridines" CHEMISTRY OF HETEROCYCLIC COMPOUNDS (A TRANSLATION OF KHIMIYAGETEROTSIKLICHESKIKH SOEDINENII), PLENUM PRESS CO., NEW YORK, NY, US LNKD- DOI:10.1007/BF00944506, vol. 9, no. 3, 1 March 1973 (1973-03-01), pages 323-326, XP008124635 ISSN: 0009-3122 [retrieved on 2005-01-13]
- BARDAMOVA M I ET AL: "Piperidol derivatives of ethynylphenols" RUSSIAN CHEMICAL BULLETIN, SPRINGER NEW YORK LLC, US; RU LNKD- DOI:10.1007/BF00852055, vol. 20, no. 3, 1 March 1971 (1971-03-01), pages 564-565, XP008124670 ISSN: 1066-5285 [retrieved on 2004-12-28]
- AZERBAEV I N ET AL: "Condensation of 1,2,5-trimethyl-4-piperidone with p-diethynylbenzene" CHEMISTRY OF HETEROCYCLIC COMPOUNDS (A TRANSLATION OF KHIMIYAGETEROTSIKLICHESKIKH SOEDINENII), PLENUM PRESS CO., NEW YORK, NY, US LNKD- DOI:10.1007/BF00522576, vol. 6, no. 12, 1 December 1970 (1970-12-01), pages 1536-1541, XP008124633 ISSN: 0009-3122 [retrieved on 2004-12-07]
- AZERBAEV I N ET AL: "Synthesis of ditertiary phenylenetetraacetylenic glycols of the piperidine series" CHEMISTRY OF HETEROCYCLIC COMPOUNDS (A TRANSLATION OF KHIMIYAGETEROTSIKLICHESKIKH SOEDINENII), PLENUM PRESS CO., NEW YORK, NY, US LNKD- DOI:10.1007/BF00945498, vol. 7, no. 5, 1 May 1971 (1971-05-01), pages 584-586, XP008124638 ISSN: 0009-3122 [retrieved on 2005-01-10]
- AZERBAEV ET AL: "Synthesis of unsymmetrical phenylenetriacetylene derivatives of 1,2,5-trimethylpiperidine" IZVESTIA AKADEMII NAUK KAZAHSKOJ SSR. SERI HIMICESKAA, NAUKA KAZSSR, ALMA-ATA, KZ, vol. 20, no. 2, 1 January 1970 (1970-01-01), pages 39-42, XP008124658 ISSN: 0002-3205
- AZERBAEV ET AL: "Synthesis and reactions of phenylenediethynylheterocyclic amino alcohols" KHIMIYA ATSETILENA I TEKHNOLOGIYA KARBIDA KAL'TSIYA -- CHEMISTRY OF ACETYLENE AND TECHNOLOGY OF CALCIUM CARBIDE, XX, KZ, 1 January 1972 (1972-01-01), pages 43-49, XP008124627
- NAZAROV I N ET AL: "Acetylene Derivatives 170. Heterocyclic compounds. XXXI Synthesis and transformations of heterocyclic acetylenic .gamma.-glycols" JOURNAL OF GENERAL CHEMISTRY USSR, CONSULTANTS BUREAU, NEW YORK, NY, US, vol. 26, 1 January 1956 (1956-01-01), pages 79-92, XP008124676 ISSN: 0022-1279
- NAZAROV I.N.: 'Heterocyclic Compounds. XLI. Synthetic Analgesic Substances. 6. Esters of 1,25-trimethyl-4-aryl-4-piperidols' ZHURNAL OBSHCHEI KHIMII vol. 26, 1956, pages 2820 - 2834, XP008122998
- NAZAROV I.N.: 'Heterocyclic Compounds. LIX. Synthetic Analgesic Substances. XX. Synthesis of Benzoic And Phenoxyacetic Esters of 1-alkenyl-2,5-dimethyl-4-ethyl-4-piperidols ' IZVESTIYA AKADEMII NAUK SSSR, SERIYA KHIMICHESKAYA 1958, pages 452 - 459, XP008119846
- HUEGI B.S.: 'Synthesis and Pharmacological Studies of 4,4-Disubstituted Piperidines: A New Class of Compounds with Potent Analgesic Properties' J. MED. CHEM. vol. 26, no. 1, 1983, pages 42 - 50, XP008119792

## Description

### TECHNICAL FIELD

This invention relates to compounds having pharmacological activity which can be used in the treatment of some conditions.

More particularly, this invention concerns certain non-imidazole tertiary amine derivatives and their salts and solvates. These compounds have H₃ histamine receptor antagonist or inverse agonist activity. This invention also relates to compounds for use in the treatment of disorders in which histamine H₃ receptor blockade is beneficial.

### BACKGROUND OF THE INVENTION

Histamine is a chemical messenger involved in various complex biological actions. When released, histamine interacts with specific macromolecular receptors on the cell surface or within a target cell to elicit changes in many different bodily functions. Various cell types including smooth muscle, blood cells, cells of the immune system, endocrine and exocrine cells as well as neurons respond to histamine by stimulating the formation of intracellular signals, including formation of phosphatidylinositol or adenylate cyclase. Evidence that histamine plays a role as a neurotransmitter was established by the mid to late 1970's (Schwartz, 1975) Life Sci. 17:503-518. Immunohistochemical studies identified histaminergic cell bodies in the tuberomammillary nucleus of the posterior hypothalamus with widespread projections in the dicencephalon and telencephalon (Inagaki et al., 1998) J. Comp. Neurol. 273:283-300.

Identification of two histamine receptors (H₁ and H₂) was reported to mediate the biochemical actions of histamine on neurons. Recently, studies have demonstrated the existence of a third subtype of histamine receptor, the histamine H₃ receptor (Schwartz et al., 1986) TIPS 8: 24-28. Various studies have now demonstrated that histamine H₃ receptors are found on the histaminergic nerve terminals in the brains of several species, including man (Arrang et al., 1983) Nature 302: 832-837. The H₃ receptor found on the histaminergic nerve terminal was defined as an autoreceptor and could intimately control the amount of histamine released from the neurons. Histamine, the natural compound, was capable of stimulating this autoreceptor but when tested against known H₁ and H₂ receptor agonists and antagonists, a distinct pharmacological profile emerged. Further, H₃ receptors have been identified on cholinergic, serotoninergic and monoamine nerve terminals in the peripheral nervous system (PNS) and central nervous system including the cerebral codex and cerebral vessels. These observations suggest that H₃ receptors are uniquely located to modulate histamine as well as other neurotransmitter release, and H₃ antagonists could be important modulators of neuronal activity.

As stated, CNS histaminergic cell bodies are found in the magnocellular nuclei of the hypothalamic mammillary region and these neurons project diffusely to large areas of the forebrain. The presence of histaminergic cell bodies in the tuberomammillary nucleus of the posterior hypothalamus, a brain area involved in the maintenance of wakefulness, and their projections to the cerebral cortex suggest a role in modulating the arousal or sleep-wake state. The histaminergic projection to many limbic structures such as the hippocampal formation and the amygdaloid complex suggest roles in functions such as autonomic regulation, control of emotions and motivated behaviors, and memory processes.

The concept that histamine is important for the state of arousal, as suggested by the location of histaminergic pathways, is supported by other types of evidence. Lesions of the posterior hypothalamus are well known to produce sleep. Neurochemical and electrophysiological studies have also indicated that the activity of histaminergic neurons is maximal during periods of wakefulness and is suppressed by barbiturates and other hypnotics. Intraventricular histamine induces the appearances of an arousal EEG pattern in rabbits and increased spontaneous locomotor activity, grooming and exploratory behavior in both saline and pentobarbital-treated rats.

In contrast, a highly selective inhibitor of histidine decarboxylase, the sole enzyme responsible for histamine synthesis, has been shown to impair waking in rats. These data support the hypothesis that histamine may function in modulating behavioral arousal. The role of the H₃ receptor in sleep-waking parameters has been recently demonstrated (Lin et al., 1990) Brain Res. 592: 325-330. Oral administration of RAMHA, a H₃ agonist, caused a significant increase in deep slow wave sleep in the cat. Conversely, thioperamide, a H₃ antagonist, enhanced wakefulness in a dose-dependent fashion. Thioperamide has also been shown to increase wakefulness and decrease slow-wave and REM sleep in rats. These findings are consistent with in vivo studies demonstrating that thioperamide caused an increase in synthesis and release of histamine. Together, these data demonstrate that selective H₃ antagonists may be useful in the treatment of arousal states and sleep disorders.

Serotonin, histamine, and acetylcholine have all been demonstrated to be diminished in the Alzheimer's (AD) brain. The histamine H₃ receptor has been demonstrated to regulate the release of each of these neurotransmitters. An H₃ receptor antagonist would therefore be expected to increase the release of these neurotransmitters in the brain. Since histamine has been demonstrated to be important in arousal and vigilance, H₃ receptor antagonists might enhance arousal and vigilance via increasing levels of neurotransmitter release and improve cognition. Thus, the use of H₃ receptor antagonists in AD, attention deficit disorders (ADD), age-related memory dysfunction and other cognitive disorders would be supported.

H₃ receptor antagonists may be useful in treating several other CNS disorders. It has been suggested that histamine may be involved in the control of sleep/wake states as well as states of arousal and alertness, cerebral circulation, energy metabolism, and hypothalmic hormone secretion. Recent evidence has indicated the possible use of H₃ antagonists in the treatment of epilepsy. Work has demonstrated an inverse correlation between the duration of clonic convulsions and brain histamine levels. Thioperamide, a H₃ antagonist, was also shown to significantly and dose-dependently decrease the durations of every convulsive phase after electrically-induced convulsions and increase the electroconvulsive threshold.

In spite of their low density, H₃ receptor binding sites can be detected outside the brain. Several studies have revealed the presence of H₃ heteroreceptors in the gastrointestinal tract, as well as upon neurons of the respitory tract. Accordingly, an H₃ receptor antagonist may be useful in the treatment of diseases and conditions such as asthma, rhinitis, airway congestion, inflammation, hyper and hypo motility and acid secretion of the gastrointestinal tract Peripheral or central blockage of H₃ receptors may also contribute to changes in blood pressure, heart rate and cardiovascular output and could be used in the treatment of cardiovascular diseases, and in the treatment of diseases or conditions such as obesity, migraine, inflammation, motion sickness, pain ADHD, dementia, depression, Parkinson's disease, schizophrenia, epilepsy, narcolepsy, acute myocardial infarction and asthma.

WO 02/12190 discloses substituted non-imidazole aryloxypiperidine compounds, compositions containing them and methods of making and using them to treat or prevent histamine-mediated conditions.

WO03/031432 discloses substituted pyperidines, pharmaceutical compositions comprising them and use thereof in the treatment of diseases and disorders related to the histidine H3 receptor.

### SUMMARY OF THE INVENTION

The present invention provides, in its principal aspect, compounds of the general formula: 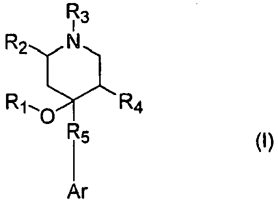 wherein
R₁ is H;
R₂, R₃ and R₄ are independently:
(C1-C8) straight or branched alkyl or (C3-C8) cycloalkyl, or
R₂ and R₃ taken together with the atoms to which they are attached form cycloalkyl -(CH₂)₃₋₇- where one or more of the methylenes may be optionally replaced by O, N or S;
R₅ is CH₂CH₂,
CH=CH,
C≡C or 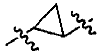 optionally substituted by (C1-C4) straight or branched alkyl or halogens; and Ar is aryl or cycloalkyl,
wherein aryl is a substituted or unsubstituted carbocyclic aromatic group having 6 to 12 carbon atoms or a heterocyclic aromatic group containing at least one endocyclic N, O or S atom and cycloalkyl is an aliphatic ring system having 3 to 8 carbon atoms and 1 to 3 rings, which can be unsubstituted or substituted with one, two or three substituents independently selected from C1-C4 alkyl, haloalkyl, alkoxy, thioalkoxy, amino, alkylamino, dialkylamino, hydroxyl, halo, mercapto, nitro, nitrite, carboxaldehyde, carboxy, alkoxycarbonyl and carboxamide, which includes cis or trans forms and may either be in endo or exo positions in the bridged bicyclic systems,
wherein alkyl is a straight or branched chain radical derived from saturated hydrocarbons by the removal of one hydrogen atom and can be unsubstituted or substituted with one, two or three substituents independently selected from C1-C4 alkyl, haloalkyl, alkoxy, thioalkoxy, amino, alkylamino, dialkylamino, hydroxyl, halo, mercapto, nitro, nitrite, carboxaldehyde, carboxy, alkoxycarbonyl and carboxamide
and the pharmaceutically acceptable salts and stereoisomers thereof, for use in treating a condition in a patient in which antagonism of histamine H₃ receptors is of therapeutic importance, said use comprising administering an effective amount of at least one said compound to a patient in need of such treatment,
wherein said condition is selected from inflammation, migraine, motion sickness, pain, Parkinson's Disease, epilepsy, cardiovascular disease, hyper or hypotension, acute myocardial infarction, gastrointestinal disorders, acid secretion, motility, CNS disorders involving attention or cognitive disorders, Alzheimer's, Attention Deficit Disorder, age-related memory dysfunction, stroke, psychiatric disorders, depression, schizophrenia, obsessive-compulsive disorders, sleep disorders, narcolepsy, sleep apnea, insomnia, disturbed biological and circadian rhythms, hyper and hypsomnolence, obesity, anorexia/bulimia, thermoregulation, and hormone release.

In another aspect, the present invention regards a compound selected from the group consisting of:
1,2,5-Trimethyl-4-quinolin-3-ylethynyl-piperidin-4-ol;
6-Methyl-7-naphthalen-1-ylethynyl-octahydro-indolizin-7-ol; and
4'-(7-Hydroxy-6-methyl-octahydro-indolizin-7-ylethynyl)-biphenyl-4-carbonitrile;
(2S,4R,5R)-l,2,5-Trimethyl-4-naphthalen-l-ylethynyl-piperidin-4-ol;
(2R,4S,5S)-1,2,5-Trimethyl-4-naphthalen-1-ylethynyl-piperidin-4-ol;
1,2,5-Trimethyl-4-(2-naphthalen-1-yl-vinyl)-piperidin-4-ol;
(2R,4S,5S)-1,2,5-Trimethyl-4-(2-naphthalen-1-yl-ethyl)-piperidin-4-ol;
1,2,5-Trimethyl-4-quinolin-5-ylethynyl-piperidin-4-ol;
4-((9-Ethyl-9H-carbazol-3-ylethynyl)-1,2,5-trimethyl-piperidin-4-ol.

### DETAILED DESCRIPTION OF THE INVENTION

The present disclosure provides compounds of the general formula: 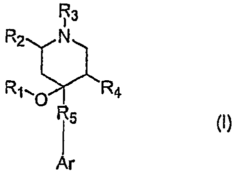 wherein
R₁ is H;
R₂, R₃ and R₄ are independently:
(C1-C8) straight or branched alkyl or (C3-C8) cycloalkyl, or
R₂ and R₃ taken together with the atoms to which they are attached form cycloalkyl -(CH₂)₃₋₇- where one or more of the methylenes may be optionally replaced by O,
N or S;
R₅ is CH₂CH₂,
CH=CH,
C≡C or 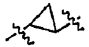 optionally substituted by (C1-C4) straight or branched alkyl or halogens; and Ar is aryl or cycloalkyl, provided that when R₂, R₃ and R₄ are straight or branched alkyl and R₅ is -C≡C-, Ar may not be unsubstituted naphthyl, and the pharmaceutically acceptable salts and stereoisomers thereof.

This disclosure also provides pharmaceutical compositions comprising a pharmaceutically acceptable carrier in combination with an effective amount of at least one compound of formula (I) 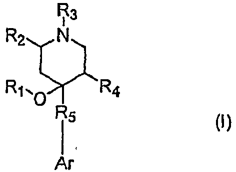 wherein
R₁ is H;
R₂, R₃ and R₄ are independently:
(C1-C8) straight or branched alkyl or (C3-C8) cycloalkyl, or
R₂ and R₃ taken together with the atoms to which they are attached form cycloalkyl -(CH₂)₃₋₇- where one or more of the methylenes may be optionally replaced by O,
N or S;
R₅ is CH₂CH₂,
CH=CH,
C≡C or 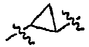 optionally substituted by (C1-C4) straight or branched alkyl or halogens; and the pharmaceutically acceptable salts and stereoisomers thereof.

The present invention provides a compound of formula (I) 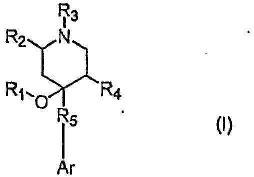 wherein
R₁ is H;
R₁ R₃ and R₄ are independently:
(C1-C8) straight or branched alkyl or (C3-C8) cycloalkyl, or
R₂ and R₃ taken together with the atoms to which they are attached form cycloalkyl -(CH₂)₃₋₇- where one or more of the methylenes may be optionally replaced by O,
NorS;
R₅ is CH₂CH₂,
CH=CH,
C≡C or 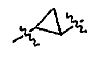 optionally substituted by (C1-C4) straight or branched alkyl or halogens; and
Ar is aryl or cycloalkyl and the pharmaceutically acceptable salts and stereoisomers thereof for use in treating conditions in which antagonism of histamine H₃ receptors is of therapeutic importance such as inflammation, migraine, motion sickness, pain, Parkinson's Disease, epilepsy, cardiovascular disease (i.e. hyper or hypotension, acute myocardial infarction), gastrointestinal disorders (acid secretion, motility) and CNS disorders involving attention or cognitive disorders (i.e., Alzheimer's, Attention Deficit Disorder, age-related memory dysfunction, stroke, etc.), psychiatric disorders (i.e., depression, schizophrenia, obsessive-compulsive disorders, etc.) and sleep disorders (i.e. narcolepsy, sleep apnea, insomnia, disturbed biological and circadian rhythms, hyper and hypsomnolence, and related disorders such as obesity, anorexia/bulimia, thermoregulation, hormone release) by administering an effective amount of a compound of formula (I) to a patient in need of such treatment.

Preferably for compounds of formula (I), R₂, R₃ and R₄ are CH₃, and R₅ is -CH₂-CH₂- or -C≡C- and Ar is 1-napthyl or

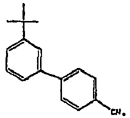

The compounds according to the invention are:
1,2,5-Trimethyl-4-quinolin-3-ylethynyl-piperidin-4-ol;
6-Methyl-7-naphthalen-1-ylethynyl-octahydro-indolizin-7-ol;
4'-(7-Hydroxy-6-methyl-octahydro-indolizin-7-ylethynyl)-biphenyl-4-carbonitrile;
(2S,4R,5R)-1,2,5-Trimethyl-4-naphthalen-1-ylethynyl-piperidin-4-ol;
(2R,4S,5S)-1,2,5-Trimethyl-4-naphthalen-1-ylethynyl-piperidin-4-ol;
1,2,5-Trimethyl-4-(2-naphthalen-1-yl-vinyl)-piperidin-4-ol;
(2R,4S,5S)-1,2,5-Trimethyl-4-(2-naphthalen-1-yl-ethyl)-piperidin-4-ol;
1,2,5-Trimethyl-4-quinolin-5-ylethynyl-piperidin-4-ol;
4-((9-Ethyl-9H-carbazol-3-ylethynyl)-1,2,5-trimethyl-piperidin-4-ol.

Certain compounds and compounds for use of the invention may exist in different isomeric (e.g. enantiomers and stereoisomers) forms. The invention contemplates all such isomers both in pure form and in a mixture, including racemic mixtures. Enol forms are also included.

The compounds and compounds for use of the invention can exist in unsolvated as well as solvated forms, including hydrated forms, e.g., hemi-hydrate. In general, the solvated forms, with pharmaceutically acceptable solvents such as water, ethanol, and the like are equivalent to the unsolvated forms for the purposes of the invention.

Certain compounds and compounds for use of the invention also form pharmaceutically acceptable salts, e.g., acid addition salts. For example, the nitrogen atoms may form salts with acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous hydroxide, potassium carbonate, ammonia, and sodium bicarbonate. The free base forms differ from their respective salt forms somewhat in certain physical properties, such as solubility in polar solvents, but the acid salts are equivalent to their respective free base forms for purposes of the invention. (See, for example S. M. Berge, et al., "Pharmaceutical Salts," J. Pharm. Sci., 66:1-19 (1977)).

As throughout this specification and appended claims, the following terms have the meanings ascribed to them:
The term "alkyl" as used herein refers to straight or branched chain radicals derived from saturated hydrocarbons by the removal of one hydrogen atom. Representative examples of alkyl groups include methyl, ethyl, n-propyl, iso-propyl, n-butyl, sec-butyl, isobutyl, tert-butyl, and the like. Alkyl groups can be unsubstituted or substituted with one, two or three substituents independently selected from lower alkyl, haloalkyl, alkoxy, thioalkoxy, amino, alkylamino, dialkylamino, hydroxyl, halo, mercapto, nitro, nitrite, carboxaldehyde, carboxy, alkoxycarbonyl and carboxamide.

The term "cycloalkyl" as used herein refers to an aliphatic ring system having 3 to 8 carbon atoms and 1 to 3 rings, including, but not limited to cyclopropyl, cyclopentyl, and cyclohexyl, among others. Cycloalkyl groups can be unsubstituted or substituted with one, two or three substituents independently selected from lower alkyl, haloalkyl, alkoxy, thioalkoxy, amino, alkylamino, dialkylamino, hydroxyl, halo, mercapto, nitro, nitrite, carboxaldehyde, carboxy, alkoxycarbonyl and carboxamide.

"Cycloalkyl" includes cis or trans forms. Furthermore, the substituents may either be in endo or exo positions in the bridged bicyclic systems.

The term "halo" or "halogen" as used herein refers to I, Br, Cl or F.

The term "aryl" or "aromatic" as used herein alone or in combination refers to a substituted or unsubstituted carbocyclic aromatic group having about 6 to 12 carbon atoms such as phenyl, naphthyl, indenyl, indanyl, azulenyl, fluorenyl and anthracenyl; or a heterocyclic aromatic group containing at least one endocyclic N, O or S atom such as furyl, thienyl, pyridyl, pyrrolyl, oxazolyl, thiazolyl, imidazolyl, pyrazolyl, 2-pyrazolinyl, pyrazolidinyl, isoxazolyl, isothiazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indolizinyl, indolyl, isoindolyl, 3H-indolyl, indolinyl, benzo[b]furanyl, 2,3-dihydrobenzofuranyl, benzo[b]thiophenyl, 1H-indazolyl, benzimidazolyl, benzthiazolyl, purinyl, 4H-quinolizinyl, isoquinolinyl, cinnolinyl, phthalazinyl, quinazolinyl, quinoxalinyl, 1,8-naphthridinyl, pteridinyl, carbazolyl, acridinyl, phenazinyl, phenothiazinyl, phenoxyazinyl, pyrazolo[1,5-c]triazinyl and the like. "Aralkyl" and "alkylaryl" employ the term "alkyl" as defined above. Rings may be multiply substituted.

The term "heteroatom" as used herein refers to at least one N, O or S atom.

As used herein, the term "composition" is intended to encompass a product comprising the specified ingredients in the specified amounts, as well as any product which results, directly or indirectly, from a combination of the specified ingredients in the specified amounts.

The compounds and compounds for use of the present invention can be used in the form of pharmaceutically acceptable salts derived from inorganic or organic acids. The phrase "pharmaceutically acceptable salt" means those salts which are, within the scope of sound medical judgment, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge et al. describe pharmaceutically acceptable salts in detail in J. Pharmaceutical Sciences,1977, 66: *1 et seg.* The salts can be prepared *in situ* during the final isolation and purification of the compounds and compounds for use of the invention or separately by reacting a free

base function with a suitable organic acid. Representative acid addition salts include, but are not limited to acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethansulfonate (isothionate), lactate, maleate, methanesulfonate, nicotinate, 2-naphthalenesulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quatemized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

Basic addition salts can be prepared *in situ* during the final isolation and purification of compounds and compounds for use of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylammonium, dimethylammonium, trimethylammonium, triethylammonium, diethylammonium, and ethylammonium among others. Other representative organic amines useful for the formation of base addition salts include ethylenediamine, ethanolamine, diethanolamine, piperidine, piperazine and the like.

Dosage forms for topical administration of a compound or a compound for use of this invention include powders, sprays, ointments and inhalants. The active compound is mixed under sterile conditions with a pharmaceutically acceptable carrier and any needed preservatives, buffers or propellants which can be required. Opthalmic formulations, eye ointments, powders and solutions are also herein disclosed.

Actual dosage levels of active ingredients in the pharmaceutical compositions of this disclosure can be varied so as to obtain an amount of the active compound(s) which is effective to achieve the desired therapeutic response for a particular patient, compositions and mode of administration. The selected dosage level will depend upon the activity of the particular compound, the route of administration, the severity of the condition being treated ) and the condition and prior medical history of the patient being treated. However, it is within the skill of the art to start doses of the compound at levels lower than required to achieve the desired therapeutic effect and to gradually increase the dosage until the desired effect is achieved.

When used in the above or other treatments, a therapeutically effective amount of one of the compounds for use of the present invention can be employed in pure form or, where such forms exist, in pharmaceutically acceptable salt, ester or prodrug form. Alternatively, the compound can be administered as a pharmaceutical composition containing the compound of interest in combination with one or more pharmaceutically acceptable excipients. The phrase "therapeutically effective amount" of the compound and compounds for use of the invention means a sufficient amount of the compound to treat disorders, at a reasonable benefit/risk ratio applicable to any medical treatment. It will be understood, however, that the total daily usage of the compounds and compounds for use of the present invention will be decided by the

attending physician within the scope of sound medical judgment. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

The total daily dose of the compounds or compounds for use of this invention administered to a human or lower animal may range from about 0.0001 to about 1000 mg/kg/day. For purposes of oral administration, more preferable doses can be in the range of from about 0.001 to about 5 mg/kg/day. If desired, the effective daily dose can be divided into multiple doses for purposes of administration; consequently, single dose compositions may contain such amounts or submultiples thereof to make up the daily dose.

The present disclosure also provides pharmaceutical compositions that comprise compounds and compounds for use of the present invention formulated togheter with one or more non-toxic pharmaceutically acceptable carriers. The pharmaceutical compositions can be specially formulated for oral administration in solid or liquid form, for parenteral injection or for rectal administration.

The pharmaceutical compositions of this disclosure can be adminitered to humans and other mammals orally, rectally, parenterally, intracisternally, intravaginally, intraperitoneally, topically (as by powders, ointments or drops), bucally or as an oral or nasal spray. The term "parenterally," as used herein, refers to modes of administration which include intravenous, intramuscular, intraperitoneal, intrasternal, subcutaneous and intraarticular injection and infusion.

In another aspect, the present disclosure provides a pharmaceutical composition comprising a component of the present invention or disclosure and a physiologically tolerable diluent. The present disclosure includes one or more compounds as described above formulated into compositions together with one or more non-toxic physiologically tolerable or acceptable diluents, carriers, adjuvants or vehicles that are collectively referred to herein as diluents, for parenteral injection, for intranasal delivery, for oral administration in solid or liquid form, for rectal or topical administration, among others.

The compositions can also be delivered through a catheter for local delivery at a target site, *via* an intracoronary stent (a tubular device composed of a fine wire mesh), or *via* a biodegradable polymer. The compounds may also be complexed to ligands, such as antibodies, for targeted delivery.

Compositions suitable for parenteral injection may comprise physiologically acceptable, sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), vegetable oils (such as olive oil), injectable organic esters such as ethyl oleate, and suitable mixtures thereof.

These compositions can also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride and the like. Prolonged absorption of the injectable pharmaceutical form can be brought about by the use of agents delaying absorption, for example, aluminum monostearate and gelatin.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

In some cases, in order to prolong the effect of the drug, it is desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Solid dosage forms for oral administration include capsules, tablets, pills, powders and granules. In such solid dosage forms, the active compound may be mixed with at least one inert, pharmaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; 0 absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills and granules can be prepared with coatings and shells such as enteric coatings and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Compositions for rectal or vaginal administration are preferably suppositories which can be prepared by mixing the compounds of this invention with suitable non-irritating excipients or carriers such as cocoa butter, polyethylene glycol or a suppositorywax which are solid at room temperature but liquid at body temperature and therefore melt in the rectum or vaginal cavity and release the active compound.

Compounds and compounds for use of the present invention can also be administered in the form of liposomes. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes can be used. The present compositions in liposome form can contain, in addition to a compound or compounds for use of the present invention, stabilizers, preservatives, excipients and the like. The preferred lipids are natural and synthetic phospholipids and phosphatidyl cholines (lecithins) used separately or together.

Methods to form liposomes are known in the art. See, for example, Prescott, Ed., Methods in Cell Biology, Volume XIV, Academic Press, New York, N.Y. (1976), p. 33 et seq*.*

The term "pharmaceutically acceptable prodrugs" as used herein represents those prodrugs of the compounds and compounds for use of the present invention which are, within the scope of sound medical judgment, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds and compounds for use of the invention. Prodrugs of the present disclosure may be rapidly transformed *in vivo* to the parent compound of the above formula, for example, by hydrolysis in blood. A thorough discussion is provided in T. Higuchi and V. Stella, Pro-drugs as Novel Delivery Systems, V. 14 of the A.C.S. Symposium Series, and in Edward B. Roche, ed., Bioreversible Carriers in Drug Design, American Pharmaceutical Association and Pergamon Press (1987).

Compounds and compounds for use of the present invention that are formed by *in vivo* conversion of a different compound that was administered to a mammal are intended to be included within the scope of the present invention.

Compounds and compounds for use of the present invention may exist as stereoisomers wherein asymmetric or chiral centers are present. These stereoisomers are "R" or "S" depending on the configuration of substituents around the chiral carbon atom. The present invention contemplates various stereoisomers and mixtures thereof. Stereoisomers include enantiomers and diastereomers, and mixtures of enantiomers or diastereomers. Individual stereoisomers of compounds and compounds for use of the present invention may be prepared synthetically from commercially available starting materials which contain asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary or (2) direct separation of the mixture of optical enantiomers on chiral chromatographic columns.

The compounds and compounds for use of the invention can exist in unsolvated as well as solvated forms, including hydrated forms, such as hemi-hydrates. In general, the solvated forms, with pharmaceutically acceptable solvents such as water and ethanol among others are equivalent to the unsolvated forms for the purposes of the invention.

The invention may be illustrated by the following representative examples.

### Example 1

### 1,2,5-Trimethyl-4-naphthalen-1-3,lethynyl-piperidin-4-ol

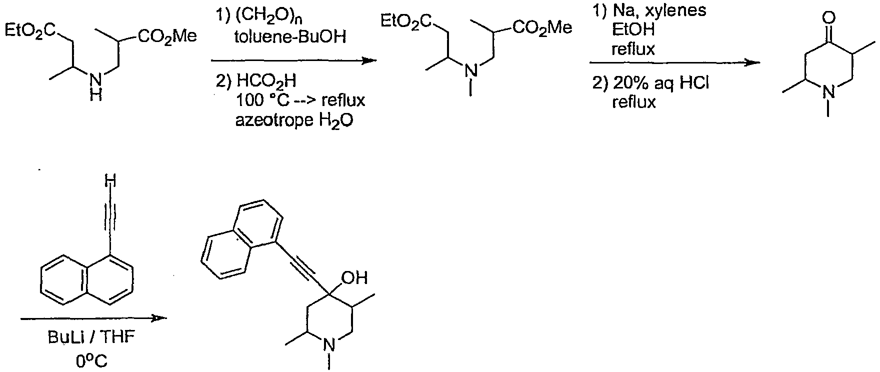

**3-[(2-Methoxycarbonyl-propyl)-methyl-amino]-butyric acid ethyl ester:** A solution of 3-(2-methoxycarbonyl-propylamino)-butyric acid ethyl ester ¹ (58.6 g, 253 mmol) and paraformaldehyde (9.9 g, 330 mmol) in toluene (250 mL) and nBuOH (25 mL) was stirred at room temperature (1 hour) followed by heating to 100 °C whereupon the reaction mixture became homogeneous. Formic acid (10 mL, 266 mmol) was dropwise over a period of 40 minutes followed by heating the reaction mixture to reflux for 45 minutes. The liberated H₂O was removed via azeotrope using a Dean-Stark apparatus. The reaction mixture was cooled to room temperature and partitioned between EtOAc (400 mL) and sat. aq. NaHCO₃ (150 mL). The aqueous layer was extracted with EtOAc (2 x 100 mL). The EtOAc layers were combined and washed with sat aq NaCl (100 mL), dried over Na₂SO₄, decanted and concentrated to give the crude product, 59.1 g (95%) that was used directly without further purification. R_{f} 0.60, 40% EtOAc-hexs, KMnO₄; visualization.
¹ Thomas, J.B.; Herault, X.M.; Rothman, R.B.; Atkinson, R.N.; Burgess, J.P.; Mascarella, S.W.; Dersch, C.M.; Xu, H.; Flippen-Anderson, J.L.; George, C.F.; Carroll, F.I. J. Med. Chem. 2001, 44, 972-987.

**1,2,5-Trimethyl-piperidin-4-one:** Sodium metal (1.1 g, 48 mmol) was added to a solution of 3-[(2-methoxycarbonyl-propyl)-methyl-amino]-butyric acid ethyl ester (59.1g) in xylenes (270 mL) at room temperature. EtOH (5 mL) was added and the resulting mixture heated to reflux for 4 hours. The reaction was cooled to ∼75 °C, at which time additional sodium metal (1.4 g, 61 mmol) was added followed by heating to refluxed for an additional 4 hours. The reaction mixture was cooled and the xylenes removed *in vacuo.* (R_{f} 0.37 10% MeOH-EtOAc, KMnO₄ visualization). 20% aq HCl was added and the mixture refluxed for 6 hours followed by cooling to 0 °C and then basified to pH 10 using 40% aq KOH. The mixture was extracted with Et₂O (6 x 100 mL). The combined Et₂O layers were washed with sat. aq. NaCl (100 mL), dried over Na₂SO₄, decanted and concentrated to give the desired product 16.01 g (47% over 3-steps). R_{f} 0.20, 10% MeOH-EtOAc, KMnO₄; visualization. The product was used without further purification.

**1,2,5-Trimethyl-4-naphthalen-1-ylethynyl-piperidin-4-ol:** BuLi (21.7 mL, 1.8 M in hex, 39 mmol) was added dropwise to a stirring solution of 1-ethynylnaphthalene (5.18 g, 5.54 mL, 39 mmol) in THF (130 mL) at 0 °C over a period of 5 minutes. The resulting solution was stirred at 0 °C for 20 minutes followed by warming to room temperature for 40 minutes. A solution of 1,2,5-trimethyl-piperidin-4-one (5.0g, 35 mmol) in THF (10 mL) was added and the resulting solution stirred at room temperature for 1 hour. The reaction mixture was then poured into cold 1 M aq HCl (100 mL) and hexanes (100 mL). The aq layer was extracted with 1:1 Et₂O-hexanes (2 x 50 mL). The aq. layer was basified to pH ∼ 9-10 using sat aq Na₂CO₃ then extracted with EtOAc (3 x 150 mL). The EtOAc layers were combined, washed with sat aq NaCl (50 mL), dried over Na₂SO₄, decanted and concentrated to give the product as a mixture of diastereomers, 6.9 g (67%). The resulting diastereomers were separated using column chromatography (SiO₂) eluting with 5% Et₃N-EtOAc. The active, and most polar, diastereomer was isolated to give, 2.26 g (22%). MS(ES): [M + 1]⁺ calculated, C₂₀H₂₄NO: 294.40; found, 294.20.

### Example 2

### Resolution of (2R,4S,5S)-1,2,5-Trimethyl-4-naphthalen-1-ylethynyl-piperidin-4-ol

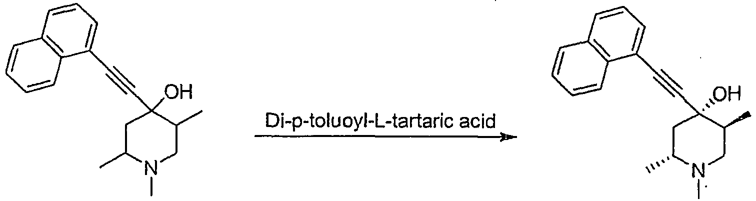

**(2R,4S,5S)-1,2,5-Trimethyl-4-naphthalen-1-ylethynyl-piperidin-4-ol:** A solution of 1,2,5-Trimethyl-4-naphthalen-1-ylethynyl-piperidin-4-ol (1.7g) and di-*p*-toluoyl-L-tartaric acid (2.24g) was prepared by addition of hot methanol to dissolve all solids. The flask was tightly sealed and allowed to cool to room temperature and stand undisturbed for 48 hours. followed by further cooling at -10°C for 24 hours in a freezer. The resultant crystallized product was isolated by vacuum filtration. The solid was washed with cold methanol and dried in a vacuum desiccator. The crystals were dissolved in water and the solution made basic (pH 10) by the addition of sodium hydroxide (10% aqueous). The solution was extracted with EtOAc (5x). The combined organic fractions were dried over MgSO₄ and concentrated under reduced pressure to give the desired enantiomer (0.522g) in greater than 95%ee (as determined by NMR of the di-*p*-toluoyl-L-tartaric acid salt). R_{f} 0.18, 5% Et₃N-EtOAc; ¹H NMR (CDCl₃, 300MHz): δ 8.32 (d, J = 8.1 Hz, 1H), 7.80-7.89 (m, 2H), 7.69 (d, J = 7.2 Hz, 1H), 7.50-7.62 (m, 2H), 7.43 (t, J = 7.7 Hz, 1H), 2.80 (dd, J₁ = 11.9 Hz, J₂ = 3.6 Hz, 1H), 2.38-2.51 (m, 1H), 2.30 (t, J = 11.9 Hz, 1H), 2.32 (s, 3H), 2.17 (dd, J₁ = 12.4 Hz, J₂ = 2.5 Hz, 1H), 2.00-2.13 (m, 1H), 1.77 (t, J = 12.1 Hz, 1H), 1.56-1.81 (br s, 1H), 1.18 (d, J = 6.2 Hz, 6H); MS (ES): [M+ 1]⁺ calculated, C₂₀H₂₄NO: 294.40; found, 294.23.
Note: A portion of the di-*p*-toluoyl-L-tartaric acid salt was crystalized for x-ray structure determination, which confirmed the stereochemical assignment above.

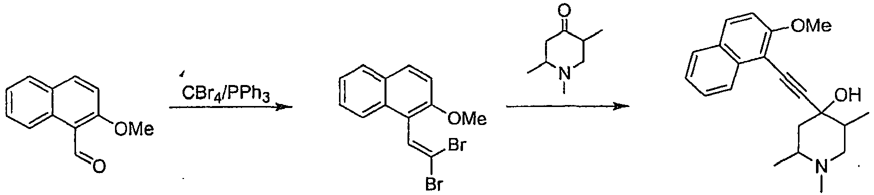

**1-(2,2-Dibromo-vinyl)-2-methoxy-naphthalene:** Triphenylphosphine (2.82 g, 10.75 mmol) and carbon tetrabromide (1.78 g, 5.37 mmol) were dissolved in CH₂Cl₂ (22 mL) and stirred for 5 minutes at 0 °C. A CH₂Cl₂ (5 mL) solution of 2-methoxy-1-naphthaldehyde was added and the resulting solution stirred at 0 °C for 15 minutes then quenched with Na₂CO₃ (25 mL, sat. aq.) and diluted with CH₂Cl₂ (25 mL). The aq layer was extracted with CH₂Cl₂ (15 mL). The combined CH₂Cl₂ layers were washed with H₂O (25 mL), sat aq NaCl (25 mL), dried over Na₂SO₄, decanted and concentrated. The resulting solid was triturated with 5% Et₂O-hexanes and filtered. The filtrate was concentrated to give the desired product, which was used in the next step without further purification.

**4-(2-Methoxy-naphthalen-1-ylethynyl)-1,2,5-trimethyl-piperidin-4-ol:** n-Butyl lithium (895 uL, 1.9 M in hexs, 1.70 mmol) was added dropwise to a solution of the dibromide (290 mg, 0.85 mmol) in THF (5.6 mL) at -78 °C. The resulting solution was warmed to room temperature over a period of 90 minutes 1,2,5-Trimethyl-piperidin-4-one (180 mg, 1.28 mmol) was added and the resulting mixture was stirred for 15 minutes at room temperature, then quenched with H₂O (15, mL) and extracted with EtOAc (3 x 25 mL). The combined EtOAc layers were washed with sat aq NaCl (25 mL), dried over Na₂SO₄, decanted and concentrated to give a mixture of diastereomers. The crude product was purified by column chromatography (SiO₂) eluting with 5% Et₃N-EtOAc to give the pure diastereomer, 44 mg, 19%. R_{f} 0.15, 5% Et₃N-EtOAc; ¹H NMR (CDCl₃, 300MHz): δ 8.23 (d, J = 8.5 Hz, 1H), 7.83 (d, J = 9.2 Hz, 1 H), 7.79 (d, J = 8.3 Hz, 1H), 7.50-7.58 (m, 1H), 7.35-7.42 (m, 1H), 7.25 (d, J = 9.2 Hz, 1H), 4.01 (s, 3H), 2.79 (dd, J₁ = 11.9 Hz, J₂ = 3.8 Hz, 1H), 2.43-2.56 (m, 1H), 2.30-2.40 (m + s (2.32), 4H), 2.18 (dd, J₁ = 12.4 Hz, J₂ = 2.6 Hz, 1H), 1.99-2.12 (m, 1H), 1.76 (t, 12.1 Hz, 1H), 1.56-1.82 (br s, 1H), 1.19 (d, J = 5.5 Hz, 3H), 1.17 (d, J = 5.1 Hz, 3H); MS (ES): [M + 1]⁺ calculated, C₂₁H₂₆NO₂: 324.43; found, 324.72.

### Example 3

### 1,2,5-Trimethyl-4-quinolin-3-ylethynyl-piperidin-4-ol

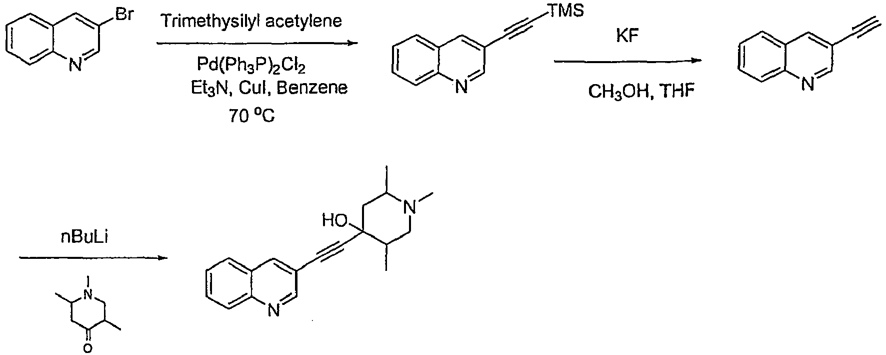

**3-Trimethylsilanylethynyl-quinoline:** To a solution of 3-bromo-quinoline (1.0 g, 5.0 mmol) in benzene (10 mL) was added triethylamine (1.4 mL, 10 mmol), copper(I) iodide (285 mg, 1.5 mmol), and trans-dichloro-bis(triphenylphosphine) palladium (350 mg, 0.5 mmol). The reaction was flushed with nitrogen for 30 seconds then trimethylsilylacetylene (1.0 mL, 7.5 mmol) added. The reaction was sealed and stirred at 70 °C overnight, cooled to room temperature and solvent removed under reduced pressure. The residue was purified via flash chromatography (10 - 30 % EtOAc/Hexane) to provide 3-trimethylsilanylethynyl-quinoline (650 mg, 56 %). MS (ES): [M + 1]⁺ calcd for C₁₄H₁₆NSi, 226.10; found, 226.76.

**3-Ethynyl-quinoline:** To a solution of 3-trimethylsilanylethynyl-quinoline (225 mg, 1.0 mmol) in methanol (1mL) and THF (1mL) was added KF (180 mg, 3.0 mmol). The mixture was stirred at room temperature for 6 hours. The solids were removed by filtration and the filtrate concentrated to give 3-Ethynyl-quinoline (150 mg, 100%). MS (ES): [M + 1]⁺ calcd for C₁₁H₈N, 154.06; found, 154.49.

**1,2,5-Trimethyl-4-quinolin-3-ylethynyl-piperidin-4-ol:** To a solution of 3-ethynyl-quinoline (153 mg, 1.0 mmol) in THF (3 mL) was added with n-butyl lithium (1.6 M, 0.63 mL, 1.0 mmol) at 0 °C. The resulting mixture was stirred at 0 °C for 5 minutes and room temperature for 20 minutes. The reaction was cooled to 0 °C and 1,2,5-trimethyl-piperidin-4-one (210 mg, 1.5 mmol) was added. The resulting mixture was stirred for 30 minutes at 0 °C, diluted with water and extracted with ethyl acetate. Organic layer was concentrated to give a residue. The residue was purified by silica gel chromatography to give the pure diastereomer (32 mg, 11%). MS (ES): [M + 1]⁺ calcd for C₁₉H₂₃N₂O, 295.17; found, 295.50. ¹H-NMR (CDCl₃, 300MHz) (ppm): δ 8.93 (s, 1H), 8.24 (s, 1H), 8.10 (d, *J*= 7.7 Hz, 1H), 7.77 - 7.78 (m, 2H), 7.57 (t, *J*= 6.2 Hz, 1H), 2.31 (s, 3H), 2.23 - 1.98 (m, 3H), 1.77 (m, 3H), 1.19 (t, *J*= 6.7 Hz, 6H).

### Example 4

### 6-Methyl-7-naphthalen-1-ylethynyl-octahydro-indolizin-7-ol

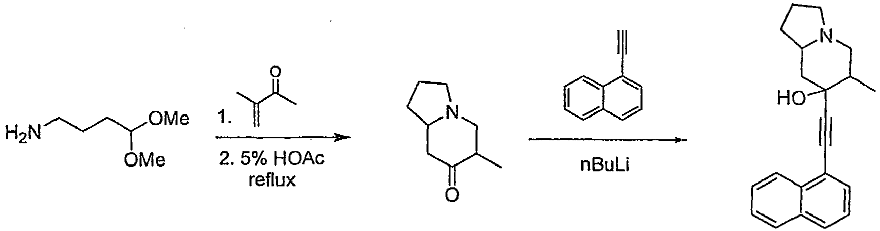

**6-Methyl-hexahydro-indolizin-7-one:** A solution of 3-methyl-3-butene-2-one (0.92g, 10.9 mmol) in methanol (2ml) was added dropwise to a cooled (0°C) solution of 4-aminobutyraldehyde dimethyl acetal (1.33g, 10mmol) in methanol (6ml). The reaction was stirred at 0°C for 30 minutes and allowed to warm to room temperature overnight. The solvent was removed under reduced pressure to give a dark yellow oil. The residue was dissolved in acetic acid (20ml, 5% aqueous) and heated to reflux for 4 hours. The reaction was cooled and the solution slowly poured into a solution of NaHCO₃ (50ml, 5% aq). The aqueous mixture was extracted with dichloromethane (4x 20ml). The combined organic fractions were washed with water, brine, dried over MgSO₄ and concentrated to give the desired product (0.72g) as a light brown oil which was used without further purification.

**6-Methyl-7-naphthalen-1-ylethynyl-octahydro-indolizin-7-ol:** n-Butyl lithium (0.6mL, 1.6 M in hex, 0.96 mmol) was added dropwise to a stirring solution of 1-ethynylnaphthalene (0.144g, 0.95 mmol) in THF (10 mL) at -78 °C. The resulting solution was stirred at -78 °C for 30 minutes A solution of 6-methyl-hexahydro-indolizin-7-one (0.072 g, 0.47mmol) in THF (3ml) was added dropwise to the reaction. The reaction was allowed to slowly warm to room temperature and stirred at room temperature for 1 hour. Water (50ml) was added to the reaction mixture the resulting solution was extracted with ethyl acetate (4x 20ml). The combined organic fractions were washed with water, brine, dried over MgSO₄ and concentrated under reduced pressure. The resulting mixture of diasteromers was separated on silica gel (5% Et₃N-EtOAc) to give the pure diastereomer, 93 mg. MS (ES): [M + 1]⁺ calcd for C₂₁H₂₄NO, 306.19; found, 306.50.

### Example 5

### 4'-(7-Hydroxy-6-methyl-octahydro-indolizin-7-ylethynyl)-biphenyl-4-carbonitrile

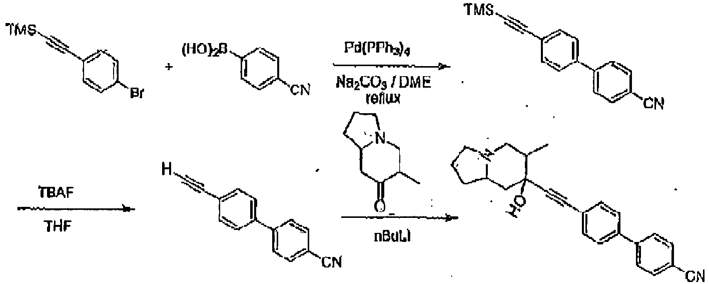

**4'-Trimethylsilanylethynyl-biphenyl-4-carbonitrile:** To a solution of (4-Bromo-phenylethynyl)-trimethyl-silane (1g, 3.95 mmol) in dimethoxyethane (10ml) was added 4-cyanophenyl boronic acid (1.2g, 8.2 mmol) palladium tetrakistriphenylphosphine (0.2g 0.17 mmol) and sodium carbonate (2ml, 2M aqueous). The reaction was flushed with nitrogen followed by heating to reflux overnight. The reaction was cooled and the resulting solution partitioned between ethyl acetate and water, The organic layer was separated and extracted with sodium hydroxide (3x 20ml, 10% aqueous), water, brine, dried over MgSO₄ and concentrated under reduced pressure. The residue was purified on silica gel (0% - 15% EtOAc:hexane) to give 4'-trimethylsilanylethynyl-biphenyl-4-carbonitrile as a light yellow solid (0.706g).

**4'-Ethynyl-biphenyl-carbonitrile:** To a solution of 4'-trimethylsilanylethynyl-biphenyl-4-carbonitrile (0.624g, 2.27mmol) in THF (5ml) and methanol (10ml) was added a solution of tetrabutylammonium fluoride (2.3ml, 2.3mmole, 1M in THF) at room temperature. The reaction was stirred at room temperature for 4 hours, TLC monitoring indicted the reaction, was complete. The reaction was partitioned between EtOAc and water. The organic layer was separated, washed with water, sat. aq. NaHCO₃, brine, dried over MgSO₄ and concentrated under reduced pressure to give the desired product as a pale yellow solid (0.531g) which was used without further purification.

**4'-(7-Hydroxy-6-methyl-octahydro-indolizin-7-ylethynyl)-biphenyl-4-carbonitrile:** n-Butyl lithium (0.66ml, 1.6M in hexane) was added dropwise to a stirring solution of 4'-Ethynyl-biphenyl-4-carbonitrile (0.2g, 0.99 mmol) in THF (5mL) at -78 °C. The resulting solution was stirred at -78 °C for 30 minutes. A solution of 6-methyl-hexahydro-indolizin-7-one (0.1 g, mmol) in THF (3ml) was added dropwise to the reaction. The reaction was allowed to slowly warm to room temperature and stirred at room temperature for 1 hour. Water (50ml) was added to the reaction mixture the resulting solution extracted with ethyl acetate (4x 20ml). The combined organic fractions were washed with water, brine, dried over MgSO₄ and concentrated under reduced pressure. The resulting mixture of diasteromers was separated on silica gel (5% Et₃N-EtOAc) to give the pure diastereomer, 35 mg. MS (ES): [M + 1]⁺ calcd for C₂₃H₂₅N₂O, 345.20; found, 345.52.

Representative compounds of the present invention that were prepared by the procedures of Examples 1-5 were evaluated in binding assays against mouse and human H₃ receptor cells by the following procedure.

### Cell culture

RAGE cells: HT1080 RAGE cells (16107.3B.B2.A8.C7) expressing the human histamine H₃ receptor and Gqαi5 were grown in alpha modified MEM containing 10% FBS, 3 ug/ml puromycin, 7 ug/ml blasticidin, and 3.2 uM methotrexate at 37 °C in 5% CO₂/95% atmosphere.

Mouse H3 cells: HT1080 (clone F5) expressing the mouse histamine H₃ receptor and Gqαi5 were grown in alpha modified MEM containing 10% FBS, 3 µg/ml puromycin, and 7 µg/ml blasticidin at 37°C in 5% CO₂/95% atmosphere.

### Membrane preparation

Cells were washed with cold PBS buffer twice, scraped off the plates, and centrifuged at 1000 x g for 5 minutes. Cells were resuspended in ice-cold buffer of 10 mM Tris, pH 7.4, 5 mM EDTA, protease inhibitor cocktail tablets (Roche Molecular Biochemicals) and incubated on ice for 10 min, homogenized with a dounce homogenizer or a polytron tissue grinder, centrifuged at 1000 x g for 10 min at 4 °C. The supernatant was centrifuged at 32,000 x g for 30 min at 4 °C. The membranes were resuspended in buffer of 50 mM Tris, pH 7.4 and protease inhibitor cocktail tablets, stored at -80 °C until use. Protein concentration was determined by the methods of Bradford.

### Radioligand binding assays

Membranes were homogenized in buffer containing 50 mM Tris/HCl, 1 mM EDTA pH 7.4, protease inhibitor cocktail tablets. Dissociation constants of radioligand (K_{D} values) and maximum binding sites (Bₘₐₓ) were determined in saturation binding experiments. Kᵢ and IC₅₀ were determined in competition assays

Binding assays were carried out in 96-well polypropylene plates in triplicate or quadruplicate. Reaction mixture contained 100 µl of membrane suspension, 50 µl of 4% DMSO, 50 µl of increasing amounts of [³H]N^{α}-methylhistamine (final concentration of 0.01-20 nM for saturation binding assay of human rage cells, 0.05-40 nM for mouse H3 cells). Nonspecific binding was defined with 10 uM clobenpropit. Competition binding assays were performed in a reaction mixture containing 100 µl of membrane suspension (∼100 µg/well for human rage H3 cells, ∼20 ug/well for mouse H3 cells), 50 µl of [³H]N^{α}-methylhistamine (final concentration is ∼2 nM for rage H3 cells and ∼10 nM for mouse H3 cells), and 50 µl compounds. Compounds were dissolved in DMSO to 10 mM and then diluted with 4% DMSO; the final maximum DMSO concentrations were 1%. Incubations were carried out for 1.5 hours at room temperature. Reactions were terminated by rapid filtration over glass fibre GF/C filters that were presoaked in 0.3% PEI for 30 minutes (Perkin Elmers, MA), using a Brandel cell harvester. The filters were washed with 500 ml of ice-cold buffer containing 50 mM Tris-HCl, pH 7.4, dried, impregnated with Meltilex wax scintillate (Perkin Elmers, MA) and counted with a Betaplate scintillation counter (Perkin Elmers, MA).

### Calcium mobilization

Cells (10⁴/well) were seeded in black 384-well plates and incubated overnight at 37°C in a 5% CO₂/95% atmosphere. After removing medium, cells were treated with CsCl Ringers buffer (136 mM CsCl, 5.4 mM KCl, 5.5 mM D-Glucose, 20 mM Hepes, pH 7.5, 2.1 mM MgCl₂, 1.2 mM CaCl₂) containing the Calcium3 dye (Molecular Device, CA) and probenecid (3.75 mM) for 60 minutes according to manufacturer's instruction. Compounds were diluted in CsCl Ringers buffer containing 0.2% bovine serum albumin and 1.0% DMSO. The concentration of R-α-methylhistamine that stimulated 75% of maximum response was used to test compounds. Ligand-induced fluorescence was measured on a Fluorometric Imaging Plate Reader (FLIPR, Molecular Device, CA).

### Data analysis

All data were analyzed by nonlinear least square curve fitting using Prism 4.0 software. The K_{D} for [³H]N^{α}-methylhistamine and the Bₘₐₓ were derived from equation RL=RₜL/(K_{D}+L). RL is concentration of receptor-bound ligand at equilibrium, L is the free ligand concentration, Rₜ is the total receptor concentration. For competition binding experiments, IC₅₀ (the concentration of compound producing 50% inhibition of specific binding) was derived from fitting to a 4-parameter logistic equation. Apparent Ki values were calculated using the Cheng-Prussof equation of Ki = IC₅₀/(1+(L)/Kd), where L is the ligand concentration. Agonist stimulation and antagonist inhibition in FLIPR were fitted to a sigmoidal dose response using equation Y=Bottom + (Top-Bottom)/(1+10^((LogEC50-X))), X is the logarithm of concentration of compounds and Y is the response.

The results of this assay are set forth in the following Table 1.

**Table 1**

| | **Chemical Name** | **Mouse H3 (uM)** | **Human H3 (uM)** |
|---|---|---|---|
| | 1,2,5-Trimethyl-4-naphthalen-1-ylethynyl-piperidin-4-ol | <1 | <0.1 |
| | (2S,4R,5R)-1,2,5-Trimethyl-4-naphthalen-1-ylethynyl-piperidin-4-ol | >1 | <0.1 |
| | (2R,4S,5S)-1,2,5-Trimethyl-4-naphthalen-1-ylethynyl-piperidin-4-ol | <0.1 | <0.01 |
| | 1,2,5-Trimethyl-4-(2-naphthalen-1-yl-ethyl)-piperidin-4-ol | <1 | <0.1 |
| | 1,2,5-Trimethyl-4-(2-naphthalen-1-yl-vinyl)-piperidin-4-ol | <1 | <0.1 |
| | (2R,4S,5S)-1,2,5-Trimethyl-4-(2-naphthalen-1-yl-ethyl)-piperidin-4-ol | <0.1 | <0.01 |
| ** | 1,3-Dimethyl-4-naphthalen-2-ylethynyl-piperidin-4-ol | >1 | >1 |
| | 1,2,5-Trimethyl-4-naphthalen-2-ylethynyl-piperidin-4-ol | <1 | <0.1 |
| | 4-(2-Butyl-quinolin-4-ylethynyl)-1,2,5-trimethyl-piperidin-4-ol | >1 | >1 |
| ** | 1,2,5-Trimethyl-4-(2-phenyl-quinolin-4-ylethynyl)-piperidin-4-ol | >1 | >1 |
| | 6-Methyl-7-naphthalen-1-ylethynyl-octahydro-indolizin-7-ol | <1 | <0.01 |
| | 1,2,5-Trimethyl-4-quinolin-5-ylethynyl-piperidin-4-ol | <1 | <0.01 |
| | 4-(9-Ethyl-9H-carbazol-3-ylethynyl)-1,2,5-trimethyl-piperidin-4-ol | <1* | |

| | | | |
|---|---|---|---|
| *Radio ligand binding data. ** comparative compounds | | | |

## Claims

1. A compound of formula 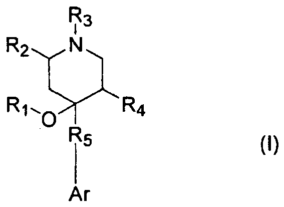 wherein
R₁ is H;
R₂, R₃ and R₄ are independently:
(C1-C8) straight or branched alkyl or (C3-C8) cycloalkyl, or
R₂ and R₃ taken together with the atoms to which they are attached form -(CH₂)₃₋₇- where one or more of the methylenes may be optionally replaced by O, NH or S;
R₅ is CH₂CH₂,
CH=CH,
C≡C or 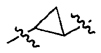 optionally substituted by (C1-C4) straight or branched alkyl or halogens; and
Ar is aryl or cycloalkyl,
wherein aryl is a substituted or unsubstituted carbocyclic aromatic group having 6 to 12 carbon atoms or a heterocyclic aromatic group containing at least one endocyclic N, O or S atom and cycloalkyl is an aliphatic ring system having 3 to 8 carbon atoms and 1 to 3 rings, which can be unsubstituted or substituted with one, two or three substituents independently selected from C1-C4 alkyl, haloalkyl, alkoxy, thioalkoxy, amino, alkylamino, dialkylamino, hydroxyl, halo, mercapto, nitro, nitrite, carboxaldehyde, carboxy, alkoxycarbonyl and carboxamide, which includes cis or trans forms and may either be in endo or exo positions in the bridged bicyclic systems,
wherein alkyl is a straight or branched chain radical derived from saturated hydrocarbons by the removal of one hydrogen atom and can be unsubstituted or substituted with one, two or three substituents independently selected from C1-C4 alkyl, haloalkyl, alkoxy, thioalkoxy, amino, alkylamino, dialkylamino, hydroxyl, halo, mercapto, nitro, nitrite, carboxaldehyde, carboxy, alkoxycarbonyl and carboxamide
and the pharmaceutically acceptable salts and stereoisomers thereof, for use in treating a condition in a patient in which antagonism of histamine H₃ receptors is of therapeutic importance, said use comprising administering an effective amount of at least one said compound to a patient in need of such treatment,
wherein said condition is selected from inflammation, migraine, motion sickness, pain, Parkinson's Disease, epilepsy, cardiovascular disease, hyper or hypotension, acute myocardial infarction, gastrointestinal disorders, acid secretion, motility, CNS disorders involving attention or cognitive disorders, Alzheimer's, Attention Deficit Disorder, age-related memory dysfunction, stroke, psychiatric disorders, depression, schizophrenia, obsessive-compulsive disorders, sleep disorders, narcolepsy, sleep apnea, insomnia, disturbed biological and circadian rhythms, hyper and hypsomnolence, obesity, anorexia/bulimia, thermoregulation, and hormone release.

2. A compound for use according to claim 1, wherein said substituted or unsubstituted carbocyclic aromatic group is selected from the group consisting of phenyl, naphthyl, indenyl, indanyl, azulenyl, fluorenyl and anthracenyl.

3. A compound for use according to claim 1, wherein said heterocyclic aromatic group is selected from the group consisting of furyl, thienyl, pyridyl, pyrrolyl, oxazolyl, thiazolyl, imidazolyl, pyrazolyl, 2-pyrazolinyl, pyrazolidinyl, isoxazolyl, isothiazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indolizinyl, indolyl, isoindolyl, 3H-indolyl, indolinyl, benzo[b]furanyl, 2,3-dihydrobenzofuranyl, benzo[b]thiophenyl, 1 H-indazolyl, benzimidazolyl, benzthiazolyl, purinyl, 4H-quinolizinyl, isoquinolinyl, cinnolinyl, phthalazinyl, quinazolinyl, quinoxalinyl, 1,8-naphthridinyl, pteridinyl, carbazolyl, acridinyl, phenazinyl, phenothiazinyl, phenoxyazinyl, pyrazolo[1,5-c]triazinyl wherein the rings may be multiply substituted.

4. A compound selected from the group consisting of:
1,2,5-Trimethyl-4-quinolin-3-ylethynyl-piperidin-4-ol;
6-Methyl-7-naphthalen-1-ylethynyl-octahydro-indolizin-7-ol;
4'-(7-Hydroxy-6-methyl-octahydro-indolizin-7-ylethynyl)-biphenyl-4-carbonitrile;
(2S,4R,5R)-1,2,5-Trimethyl-4-naphthalen-1-ylethynyl-piperidin-4-ol;
(2R,4S,5S)-1,2,5-Trimethyl-4-naphthalen-1-ylethynyl-piperidin-4-ol;
1,2,5-Trimethyl-4-(2-naphthalen-1-yl-vinyl)-piperidin-4-ol;
(2R,4S,5S)-1,2,5-Trimethyl-4-(2-naphthalen-1-yl-ethyl)-piperidin-4-ol;
1,2,5-Trimethyl-4-quinolin-5-ylethynyl-piperidin-4-ol;
4-((9-Ethyl-9H-carbazol-3-ylethynyl)-1,2,5-trimethyl-piperidin-4-ol.

## Patentansprüche

1. Eine Verbindung mit der Formel 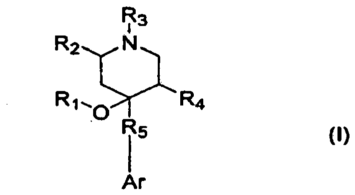 worin
R₁ H ist;
R₂, R₃ und R₄ sind unabhängig:
(C1-C8) gerades oder verzweigtes Alkyl oder (C3-C8) Cycloalkyl, oder
R₂ und R₃ bilden zusammengenommen mit den Atomen, an welche sie gebunden sind, -(CH₂)₃₋₇-, worin eines oder mehrere der Methylene wahlweise ersetzt werden kann durch O, NH oder S,
R₅ ist CH₂CH₂,
CH=CH,
C=C oder 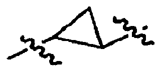 wahlweise substituiert durch (C1-C4) gerades oder verzweigtes Alkyl oder Halogene; und
Ar ist Aryl oder Cycloalkyl,
worin Aryl eine substituierte oder unsubstituierte carbozyklische aromatische Gruppe ist mit 6 bis 12 Kohlenstoffatomen, oder eine heterozyklische aromatische Gruppe enthaltend mindestens ein endozyklisches N, O oder S Atom, und Cycloalkyl ist ein aliphatisches Ringsystem, das 3 bis 8 Kohlenstoffatome und 1 bis 3 Ringe hat, welche unsubstituiert oder substituiert sein können mit einem, zwei oder drei Substituenten unabhängig gewählt aus C1-C4 Alkyl, Haloalkyl, Alkoxy, Thioalkoxy, Amino, Alkylamino, Dialkylamino, Hydroxyl, Halo, Mercapto, Nitro, Nitrit, Carboxaldehyd, Carboxy, Alkoxycarbonyl und Carboxamid, was cis- oder trans-Formen einschließt, und sie können entweder in endo- oder exo-Positionen in den verbrückten bizyklischen Systemen vorliegen,
worin Alkyl ein gerades oder verzweigtes Kettenradikal ist, abgeleitet von gesättigten Kohlenwasserstoffen durch das Entfernen von einem Wasserstoffatom, und es kann unsubstituiert oder substituiert sein mit einem, zwei oder drei Substituenten unabhängig gewählt aus C1-C4 Alkyl, Haloalkyl, Alkoxy, Thioalkoxy, Amino, Alkylamino, Dialkylamino, Hydroxyl, Halo, Mercapto, Nitro, Nitrit, Carboxaldehyd, Carboxy, Alkoxycarbonyl und Carboxamid,
und die pharmazeutisch verträglichen Salze und Stereoisomere davon, für die Verwendung in der Behandlung eines Zustandes in einem Patienten, in dem der Antagonismus von Histamin H₃ Rezeptoren von therapeutischer Wichtigkeit ist, wobei die Verwendung das Verabreichen einer wirksamen Menge von mindestens einer der Verbindung an einen Patienten, der eine solche Behandlung benötigt, umfasst,
worin der Zustand gewählt ist aus Entzündung, Migräne, Reisekrankheit, Schmerz, Parkinson'scher Krankheit, Epilepsie, kardiovaskulärer Erkrankung, Hyper- oder Hypotonie, akutem Myokardinfarkt, Magen-Darm-Erkrankungen, Säuresekretion, Motilität, ZNS-Störungen, die Aufmerksamkeits- oder kognitive Störungen einschließen, Alzheimer'scher Krankheit, Aufmerksamkeitsdefizitstörung, altersbedingter Gedächtnisstörung, Schlaganfall, psychiatrischen Erkrankungen, Depression, Schizophrenie, Zwangsstörungen, Schlafstörungen, Narkolepsie, Schlafapnoe, Schlaflosigkeit, gestörten biologischen und zirkadianen Rhythmen, Hyper- und Hypsomnolenz, Fettleibigkeit, Anorexie/Bulimie, Thermoregulation und Hormonausschüttung.

2. Eine Verbindung für die Verwendung gemäß Anspruch 1, worin die substituierte oder unsubstituierte carbozyklische aromatische Gruppe gewählt ist aus der Gruppe bestehend aus Phenyl, Naphthyl, Indenyl, Indanyl, Azulenyl, Fluorenyl und Anthracenyl.

3. Eine Verbindung für die Verwendung gemäß Anspruch 1, worin die heterozyklische aromatische Gruppe gewählt ist aus der Gruppe bestehend aus Furyl, Thienyl, Pyridyl, Pyrrolyl, Oxazolyl, Thiazolyl, Imidazolyl, Pyrazolyl, 2-Pyrazolinyl, Pyrazolidinyl, Isoxazolyl, Isothiazolyl, 1,2,3-Oxadiazolyl, 1,2,3-Triazolyl, 1,3,4-Thiadiazolyl, Pyridazinyl, Pyrimidinyl, Pyrazinyl, 1,3,5-Triazinyl, 1,3,5-Trithianyl, Indolizinyl, Indolyl, Isoindolyl, 3H-Indolyl, Indolinyl, Benzo[b]furanyl, 2,3-Dihydrobenzofuranyl, Benzo[b]thiophenyl, 1H-Indazolyl, Benzimidazolyl, Benzthiazolyl, Purinyl, 4H-Chinolizinyl, Isochinolinyl, Cinnolinyl, Phthalazinyl, Chinazolinyl, Chinoxalinyl, 1,8-Naphthridinyl, Pteridinyl, Carbazolyl, Acridinyl, Phenazinyl, Phenothiazinyl, Phenoxyazinyl, Pyrazolo[1,5-c]triazinyl, worin die Ringe mehrfach substituiert sein können.

4. Eine Verbindung gewählt aus der Gruppe bestehend aus:
1,2,5-Trimethyl-4-chinolin-3-ylethinyl-piperidin-4-ol;
6-Methyl-7-naphthalen-1-ylethinyl-octahydro-indolizin-7-ol;
4'-(7-Hydroxy-6-methyl-octahydro-indolizin-7-ylethinyl)-biphenyl-4-carbonitril;
(2S,4R,5R)-1,2,5-Trimethyl-4-naphthalen-1-ylethinyl-piperidin-4-ol;
(2R,4S,5S)-1,2,5-Trimethyl-4-naphthalen-1-ylethinyl-piperidin-4-ol;
1,2,5-Trimethyl-4-(2-naphthalen-1-yl-vinyl)-piperidin-4-ol;
(2R,4S,5S)-1,2,5-Trimethyl-4-(2-naphthalen-1-yl-ethyl)-piperidin-4-ol;
1,2,5-Trimethyl-4-chinolin-5-ylethinyl-piperidin-4-ol;
4-((9-Ethyl-9H-carbazol-3-ylethinyl)-1,2,5-trimethyl-piperidin-4-ol.

## Revendications

1. Composé de formule 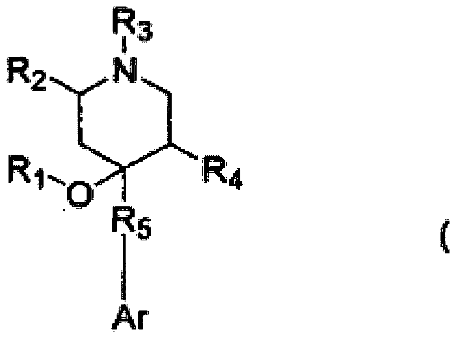 dans laquelle
R₁ est H ;
R₂, R₃ et R₄ sont indépendamment :
un groupe alkyle en C₁ à C₈ linéaire ou ramifié ou
un groupe cycloalkyle en C₃ à C₈, ou
R₂ et R₃ pris ensemble avec les atomes auxquels ils sont attachés forment -(CH₂)₃₋₇- où un ou plusieurs des groupes méthylène peuvent être facultativement remplacés par O, NH ou S;
R₅ est CH₂CH₂,
CH=CH,
C=C ou 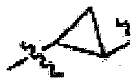 facultativement substitué par un groupe alkyle en C₁ à C₄ linéaire ou ramifié ou des atomes d'halogène ; et
Ar est un groupe aryle ou cycloalkyle,
où le groupe aryle est un groupe aromatique carbocyclique substitué ou non substitué ayant de 6 à 12 atomes de carbone ou un groupe aromatique hétérocyclique contenant au moins un atome N, O ou S endocyclique et le groupe cycloalkyle est un système de cycles aliphatique ayant de 3 à 8 atomes de carbone et de 1 à 3 cycles, qui peut être non substitué ou substitué par un, deux ou trois substituants indépendamment choisis parmi les groupes alkyle en C₁ à C₄, halogénoalkyle, alcoxy, thioalcoxy, amino, alkylamino, dialkylamino, hydroxyle, halogéno, mercapto, nitro, nitrite, carboxaldéhyde, carboxy, alcoxycarbonyle et carboxamide, qui comprend les formes cis ou trans et peut être en positions endo ou exo dans les systèmes bicycliques pontés,
où le groupe alkyle est un radical à chaîne linéaire ou ramifiée dérivé d'hydrocarbures saturés par l'élimination d'un atome d'hydrogène et peut être non substitué ou substitué par un, deux ou trois substituants indépendamment choisis parmi les groupes alkyle en C₁ à C₄, halogénoalkyle, alcoxy, thioalcoxy, amino, alkylamino, dialkylamino, hydroxyle, halogéno, mercapto, nitro, nitrite, carboxaldéhyde, carboxy, alcoxycarbonyle et carboxamide,
et les sels pharmaceutiquement acceptables et les stéréoisomères de celui-ci, pour une utilisation dans le traitement d'une pathologie chez un patient chez lequel l'antagonisme des récepteurs de l'histamine H₃ est d'importance thérapeutique, ladite utilisation comprenant l'administration d'une quantité efficace d'au moins un dudit composé à un patient ayant besoin d'un tel traitement,
dans lequel ladite pathologie est choisie parmi l'inflammation, la migraine, le mal des transports, la douleur, la maladie de Parkinson, l'épilepsie, les maladies cardiovasculaires, l'hyper ou l'hypotension, l'infarctus du myocarde aigu, les troubles gastro-intestinaux, la sécrétion acide, la motilité, les troubles du SNC impliquant les troubles cognitifs ou de l'attention, la maladie d'Alzheimer, le trouble déficitaire de l'attention, le dysfonctionnement de la mémoire lié à l'âge, l'AVC, les troubles psychiatriques, la dépression, la schizophrénie, les troubles obsessifs-compulsifs, les troubles du sommeil, la narcolepsie, l'apnée du sommeil, l'insomnie, les rythmes biologiques et circadiens perturbés, l'hyper et l'hyposomnolence, l'obésité, l'anorexie/boulimie, la thermorégulation et la libération d'hormones.

2. Composé pour une utilisation selon la revendication 1, dans lequel ledit groupe aromatique carbocyclique substitué ou non substitué est choisi dans le groupe constitué par les groupes phényle, naphtyle, indényle, indanyle, azulényle, fluorényle et anthracényle.

3. Composé pour une utilisation selon la revendication 1, dans lequel ledit groupe aromatique hétérocyclique est choisi dans le groupe constitué par les groupes furyle, thiényle, pyridyle, pyrrolyle, oxazolyle, thiazolyle, imidazolyle, pyrazolyle, 2-pyrazolinyle, pyrazolidinyle, isoxazolyle, isothiazolyle, 1,2,3-oxadiazolyle, 1,2,3-triazolyle, 1,3,4-thiadiazolyle, pyridazinyle, pyrimidinyle, pyrazinyle, 1,3,5-triazinyle, 1,3,5-trithianyle, indolizinyle, indolyle, isoindolyle, 3H-indolyle, indolinyle, benzo[b]furanyle, 2,3-dihydrobenzofuranyle, benzo[b]thiophényle, 1H-indazolyle, benzimidazolyle, benzthiazolyle, purinyle, 4H-quinolizinyle, isoquinoléinyle, cinnolinyle, phtalazinyle, quinazolinyle, quinoxalinyle, 1,8-naphtridinyle, ptéridinyle, carbazolyle, acridinyle, phénazinyle, phénothiazinyle, phénoxyazinyle, pyrazolo[1,5-c]triazinyle, dans lequel les cycles peuvent substitués de multiples fois.

4. Composé choisi dans le groupe constitué par :
1,2,5-triméthyl-4-quinoléin-3-yléthynyl-pipéridin-4-ol ;
6-méthyl-7-naphtalén-1-yléthynyl-octahydro-indolizin-7-ol ;
4'-(7-hydroxy-6-méthyl-octahydro-indolizin-7-yléthynyl)-biphényl-4-carbonitrile ;
(2S,4R,5R)-1,2,5-triméthyl-4-naphtalén-1-yléthynyl-pipéridin-4-ol ;
(2R,4S,5S)-1,2,5-triméthyl-4-naphtalén-1-yléthynyl-pipéridin-4-ol ;
1,2,5-triméthyl-4-(2-naphtalén-1-yl-vinyl)-pipéridin-4-ol ;
(2R,4S,5S)-1,2,5-triméthyl-4-(2-naphtalén-1-yl-éthyl)-pipéridin-4-ol ;
1,2,5-triméthyl-4-quinoléin-5-yléthynyl-pipéridin-4-ol ;
4-((9-éthyl-9H-carbazol-3-yléthynyl)-1,2,5-triméthyl-pipéridin-4-ol.
